(19) Europäisches Patentamt
European Patent Office
Office européen des brevets

(11) EP 4 621 010 A1

(12) EUROPEAN PATENT APPLICATION

(43) Date of publication:
**24.09.2025 Bulletin 2025/39**

(21) Application number: **25173141.0**

(22) Date of filing: **22.02.2017**

(51) International Patent Classification (IPC):
***C08L 69/00*** *(2006.01)*

(52) Cooperative Patent Classification (CPC):
(C-Sets available)
**C08L 69/00;** C08L 2205/025 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **26.02.2016 EP 16382081**
**21.12.2016 EP 16382639**

(62) Document number(s) of the earlier application(s) in accordance with Art. 76 EPC:
**17707962.1 / 3 420 030**

(71) Applicant: **SABIC Global Technologies B.V.**
**4612 PX Bergen op Zoom (NL)**

(72) Inventors:
- **Del Agua Hernandez, David**
  **6160 GA Geleen (NL)**
- **Martinez Canovas, Maria Dolores**
  **6160 GA Geleen (NL)**
- **van de Grampel, Robert Dirk**
  **6160 GA Geleen (NL)**

(74) Representative: **Sabic Intellectual Property Group**
**Sabic Intellectual Property Department**
**P.O. Box 3008**
**6160 GA Geleen (NL)**

Remarks:
This application was filed on 29-04-2025 as a divisional application to the application mentioned under INID code 62.

(54) **IMPACT PERFORMANCE MODIFIED MELT POLYCARBONATE**

(57) The present disclosure relates to articles formed from composition comprising a melt polycarbonate component and an interfacial polycarbonate component. The resulting articles exhibit mechanical properties improving upon articles formed from polycarbonate prepared according to conventional melt polymerization processes.

(52) Cooperative Patent Classification (CPC): (Cont.)

C-Sets
**C08L 69/00, C08L 69/00**

## Description

### TECHNICAL FIELD

The disclosure concerns methods for producing polycarbonate according to melt polymerization processes.

### BACKGROUND

Polycarbonates (PC) are used in a number of industries for a variety of applications owing to their versatility and desirable properties. Polycarbonates provide high transparency and are esteemed for high impact and high heat resistance. Polycarbonates are generally produced according to one of two commercial production methods: a two-phase interfacial process and a melt transesterification process. The interfacial method comprises the reaction of at least one dihydroxy compound, generally a di-hydroxyaromatic compound, with phosgene in a solvent, in the presence of a basic reagent as acid acceptor and an amine as catalyst. Melt transesterification processes are well known in the art for producing polycarbonate by reacting a diaryl carbonate and a dihydroxy compound in the optional presence of transesterification catalysts. Other potentially useful methods tend to be direct variations on, or simple combinations of, these two primary processes.

Polycarbonates are transparent engineering thermoplastics with improved impact properties compared with other transparent polymers such as polymethyl methacrylate or polybutylene terephthalate. However, at low temperature the polycarbonate resin may present a brittle behavior and the impact properties of the material may not be sufficient to fulfill certain application requirements.

In order to avoid these problems, several types of additives may be blended with the polycarbonate resin. The use of additives such as elastomers (ABS or MBS) or reinforcing fibers it is a well kwon strategy. Such reinforcing additives are generally blended with the polycarbonate during the formulation step and, unfortunately, the polycarbonate becomes opaque.

U.S. Pat. Nos. 5,504,177 and 6,066,700 purport to describe the use of siloxanes to produce transparent materials having good impact performance at low temperature. The copolymerization strategy described therein adds an extra cost and complexity due to the lower reactivity of the carbonate group usage (as in diphenyl carbonate, DPC).

U.S. Pat. No. and 5,959,065 purports to describe the use of "high volume" end cappers in the polycarbonate chain to improve the impact properties of a polycarbonate material without any negative impact in the transparency of the material. Nevertheless, this strategy increases the cost of the resin due to the addition of a new monomer and, in the particular case of the polycarbonate obtained by the melt process, it is difficult to modify the process to add this extra monomer.

These and other shortcomings of the prior art are addressed by the present disclosure.

### SUMMARY

In the present disclosure, an improvement in the impact properties of polycarbonate (PC) melt resin for low viscosity grades with melt volume rate (MVR) from about 20 $cm^3$/min (1.2 Kg- 300°C ISO 1133) to about 30 $cm^3$/min is shown. This improvement has been achieved by blending the melt PC resin with other PC interfacial resin. The interfacial PC resin component has a different type of endcap compared with traditional endcaps groups of the melt PC resin. The endcap groups of the interfacial PC resin are based on the use of bulky molecules (phenol, phosgene, p-cumyl phenol, p-tertbutyl phenol) compared with typical endgroups (BPA) present in the melt resin.

The increasing of the bulky endgroups concentration by adding different proportions of one PC interfacial grade based on 100 % p-cumyl phenol end cap to a melt PC resin improves the impact performance of the melt material compared with the same resin without the interfacial polycarbonate. As an example, such an addition can increase the bulkiness of the endgroups without increasing -OH (e.g., reactivity).

While aspects of the present disclosure may be described and claimed in a particular statutory class, such as the system statutory class, this is for convenience only and one of skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Additional aspects of the disclosure will be set forth in part in the description which follows, and in part will be obvious from the description, or can be learned by practice of the disclosure. The advantages of the disclosure will be realized and attained by means of the elements and combinations particularly pointed out in the appended claims. It is to be

understood that both the foregoing general description and the following detailed description are exemplary and explanatory only and are not restrictive of the disclosure, as claimed.

## BRIEF DESCRIPTION OF FIGURES

FIG. 1 presents a plot of the impact performance of the blends of interfacial and melt polycarbonate at different temperatures.
FIG. 2 presents the impact performance as a function of temperature for blends with varying amounts of interfacial and melt polycarbonate.
FIG. 3 presents the impact performance as a function of the percent content of bulky endcapping groups in the interfacial and melt polycarbonate blends.
FIG. 4 presents the impact performance as a function of the interfacial polycarbonate present in the blend at 10 °C.
FIG. 5 impact performance as a function of the interfacial polycarbonate present in the blend at 5 °C.
FIG. 6 impact performance as a function of the interfacial polycarbonate present in the blend at 0 °C.
FIG. 7 impact performance as a function of the interfacial polycarbonate present in the blend at -1 °C.

## DETAILED DESCRIPTION

While "high volume" end cappers may be used to improve the impact properties of the polycarbonate without a negative impact on transparency, these high volume end cappers are primarily used in interfacial polymerization processes. Though siloxane additives may be used in an interfacial polymerization process to provide transparent polycarbonate with good impact performance at low temperatures, siloxanes may add an undue cost and are not easily applied in melt polycarbonate polymerization processes (lower reactivity of the carbonate source, diphenyl carbonate). Accordingly, a melt polycarbonate resin exhibiting properties comparable to an interfacial polycarbonate resin would be desirable.

Generally, in a melt polymerization process, polycarbonates may be prepared by co-reacting, in a molten state, a dihydroxy compound (s) and a carbonate source, such as diphenyl carbonate, or more specifically in an aspect, an activated carbonate such as bis(methyl salicyl)carbonate, in the presence of a transesterification catalyst. In the melt polymerization process, the reaction mixture may comprise a melt transesterification catalyst, a dihydroxy compound, a carbonate source and a phenolic byproduct. More specifically, the polycarbonate may be produced by the melt transesterification reaction of a dihydroxy compound and diphenyl carbonate in the presence of the melt transesterification catalyst. As the reaction proceeds, the diphenyl carbonate may be consumed while the phenolic byproduct is generated.

In an aspect, the melt polymerization reaction mixture may comprise a melt transesterification catalyst. Melt transesterification catalysts are well-known in the art and are not limited to the examples disclosed herein. Exemplary melt transesterification catalysts are disclosed in U.S. Patent Nos. 7,365,149, 7,547,799, 7,619,053, and 7,671, 165.

In some aspects, the melt transesterification catalyst may include at least one alpha and/or beta transesterification catalyst. The alpha catalyst, or the first catalyst, may typically be more thermally stable and less volatile than the beta, or second, catalyst. As such, an alpha catalyst may be more useful to the melt polymerization reaction if used in later high-temperature polymerization stages. In various aspects, the alpha catalyst may comprise metal or ions (cations or anions). In further examples, the alpha catalyst may comprise a metal cation and an anion. In a specific example, the cation may be an alkali or alkaline earth metal comprising Li, Na, K, Cs, Rb, Mg, Ca, Ba, Sr, or a combination comprising at least one of the foregoing. The anion may be a hydroxide ($OH^-$), superoxide ($O^{2-}$), thiolate ($HS^-$), sulfide ($S^{2-}$), a $C_{1-20}$ alkoxide, a $C_{6-20}$ aryloxide, a $C_{1-20}$ carboxylate, a phosphate including biphosphate, a $C_{1-20}$ phosphonate, a sulfate including bisulfate, sulfites including bisulfites and metabisulfites, a $C_{1-20}$ sulfonate, a carbonate including bicarbonate, or a combination comprising at least one of the foregoing. In another aspect, salts of an organic acid comprising both alkaline earth metal ions and alkali metal ions may also be used. Salts of organic acids useful as catalysts are illustrated by alkali metal and alkaline earth metal salts of formic acid, acetic acid, stearic acid and ethyelenediaminetetraacetic acid (EDTA). The catalyst may also comprise the salt of a non-volatile inorganic acid. By "nonvolatile", it is meant that the referenced compounds have no appreciable vapor pressure at ambient temperature and pressure. In particular, these compounds are not volatile at temperatures at which melt polymerizations of polycarbonate are typically conducted. The salts of nonvolatile acids are alkali metal salts of phosphites; alkaline earth metal salts of phosphites; alkali metal salts of phosphates; and alkaline earth metal salts of phosphates.

Exemplary alpha transesterification catalysts include, lithium hydroxide, sodium hydroxide, potassium hydroxide, cesium hydroxide, magnesium hydroxide, calcium hydroxide, barium hydroxide, lithium formate, sodium formate, potassium formate, cesium formate, lithium acetate, sodium acetate, potassium acetate, lithium carbonate, sodium carbonate, potassium carbonate, lithium methoxide, sodium methoxide, potassium methoxide, lithium ethoxide, sodium

ethoxide, potassium ethoxide, lithium phenoxide, sodium phenoxide, potassium phenoxide, sodium sulfate, potassium sulfate, $NaH_2PO_3$, $NaH_2PO_4$, $Na_2H_2PO_3$, $KH_2PO_4$, $CsH_2PO_4$, $Cs_2H_2PO_4$, $Na_2SO_3$, $Na_2S_2O_5$, sodium mesylate, potassium mesylate, sodium tosylate, potassium tosylate, magnesium disodium ethylenediamine tetraacetate (EDTA magnesium disodium salt), or a combination comprising at least one of the foregoing. It will be understood that the foregoing list is exemplary and should not be considered as limited thereto.

In one aspect, the alpha transesterification catalyst is an alpha catalyst comprising an alkali or alkaline earth salt. In an exemplary aspect, the transesterification catalyst may comprise sodium hydroxide, potassium hydroxide, sodium carbonate, potassium carbonate, sodium methoxide, potassium methoxide, $NaH_2PO_4$, or a combination comprising at least one of the foregoing.

The amount of alpha catalyst may vary widely according to the conditions of the melt polymerization, and may be about 0.001 micromole ($\mu$mol) to about 500 $\mu$mol. In further aspects, the amount of beta catalyst used may be based upon the total number of moles of dihydroxy compound used in the melt polymerization reaction.

In another aspect, the beta catalyst, i.e., a second transesterification catalyst, may optionally be included in the melt polymerization process, provided that the inclusion of such a second transesterification catalyst does not significantly adversely affect the desirable properties of the polycarbonate. A beta catalyst may include a quaternary ammonium compound, a quaternary phosphonium compound, or a combination comprising at least one of the foregoing. Exemplary transesterification catalysts may further include a combination of a phase transfer catalyst of formula $(R^3)_4Q^+X$ above, wherein each $R^3$ is the same or different, and is a $C_{1-10}$ alkyl group; Q is a nitrogen or phosphorus atom; and X is a halogen atom or a $C_{1-8}$ alkoxy group or $C_{6-18}$ aryloxy group. Exemplary phase transfer catalyst salts include, for example, $[CH_3(CH_2)_3]_4NX$, $[CH_3(CH_2)_3]_4PX$, $[CH_3(CH_2)_5]_4NX$, $[CH_3(CH_2)_6]_4NX$, $[CH_3(CH_2)_4]_4NX$, $CH_3[CH_3(CH_2)_3]_3NX$, and $CH_3[CH_3(CH_2)_2]_3NX$, wherein X is $Cl^-$, $Br^-$, a $C_{1-8}$ alkoxy group or a $C_{6-18}$ aryloxy group.

Examples of such beta transesterification catalysts include tetrabutylammonium hydroxide, methyltributylammonium hydroxide, tetrabutylammonium acetate, tetrabutylphosphonium hydroxide, tetrabutylphosphonium acetate, tetrabutylphosphonium phenolate, or a combination comprising at least one of the foregoing. Other melt transesterification catalysts include alkaline earth metal salts or alkali metal salts. In various aspects, where a beta catalyst is desired, the beta catalyst may be present in a molar ratio, relative to the alpha catalyst, of less than or equal to 10, specifically less than or equal to 5, more specifically less than or equal to 1, and still more specifically less than or equal to 0.5. In other aspects, the melt polymerization reaction disclosed herein uses only an alpha catalyst as described hereinabove, and is substantially free of any beta catalyst. As defined herein, "substantially free of" may mean where the beta catalyst has been excluded from the melt polymerization reaction. In one aspect, the beta catalyst is present in an amount of less than about 10 ppm, specifically less than 1 ppm, more specifically less than about 0.1 ppm, more specifically less than or equal to about 0.01 ppm, and more specifically less than or equal to about 0.001 ppm, based on the total weight of all components used in the melt polymerization reaction.

In various aspects, the melt polymerization process disclosed herein may comprise a dihydroxy compound. Dihydroxy compounds of the present disclosure may have the formula $HO\text{-}R^1\text{-}OH$, which includes dihydroxy compounds of formula (1):

$$HO\text{-}A^1\text{-}Y^1\text{-}A^Z\text{-}OH \qquad (1),$$

wherein $Y^1$, $A^1$ and $A^2$ are as described above. Also included are bisphenol compounds of general formula (2):

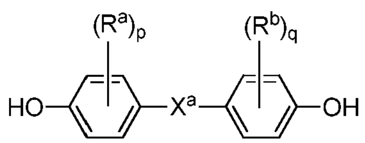
(2),

wherein $R^a$ and $R^b$ each represent a halogen atom or a monovalent hydrocarbon group and may be the same or different; p and q are each independently integers from 0 to 4; and $X^a$ represents one of the groups of formula (3):

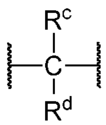
or
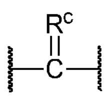
(3),

wherein $R^c$ and $R^b$ each independently represent a hydrogen atom or a monovalent linear or cyclic hydrocarbon group and $R^e$ is a divalent hydrocarbon group.

In various aspects, examples of suitable dihydroxy compounds include the dihydroxy-substituted hydrocarbons

disclosed by name or formula (generic or specific) in U.S. Pat. No. 4,217,438. A nonexclusive list of specific examples of suitable dihydroxy compounds includes the following: resorcinol, 4-bromoresorcinol, hydroquinone, 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1-naphthylmethane, 1,2-bis(4-hydroxyphenyl)ethane, 1,1-bis(4-hydroxyphenyl)-1-phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis (hydroxyphenyl)cyclopentane, 1,1-bis(4-hydroxyphenyl)cyclohexane, 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantine, (alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'-tetramethylspiro(bis)indane ("spirobiindane bisphenol"), 3,3-bis(4-hydroxyphenyl)phthalide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, 2,7-dihydroxycarbazole, 3,3-bis(4-hydroxyphenyl)phthalimidine, 2-phenyl-3,3-bis-(4-hydroxyphenyl)phthalimidine (PPPBP), and the like, as well as mixtures including at least one of the foregoing dihydroxy compounds.

In a further aspect, examples of the types of bisphenol compounds that may be represented by formula (3) includes 1,1-bis(4-hydroxyphenyl)methane, 1,1-bis(4-hydroxyphenyl)ethane, 2,2-bis(4-hydroxyphenyl)propane (hereinafter "bisphenol A" or "BPA"), 2,2-bis(4-hydroxyphenyl)butane, 2,2-bis(4-hydroxyphenyl)octane, 1,1-bis(4-hydroxyphenyl)propane, 1,1-bis(4-hydroxyphenyl) n-butane, 2,2-bis(4-hydroxy-1-methylphenyl)propane, and 1,1-bis(4-hydroxy-t-butylphenyl)propane. Combinations including at least one of the foregoing dihydroxy compounds may also be used. In various further aspects, bisphenols containing substituted or unsubstituted cyclohexane units may be used, for example bisphenols of formula (4):

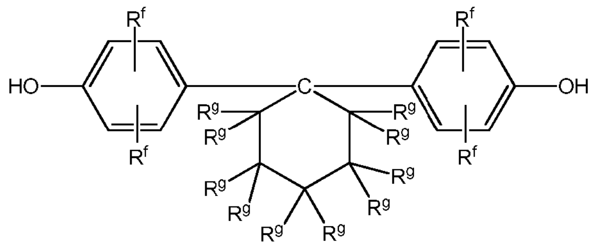

(4)

wherein each $R^f$ is independently hydrogen, $C_{1-12}$ alkyl, or halogen; and each $R^g$ is independently hydrogen or $C_{1-12}$ alkyl. The substituents may be aliphatic or aromatic, straight chain, cyclic, bicyclic, branched, saturated, or unsaturated. Such cyclohexane-containing bisphenols, for example the reaction product of two moles of a phenol with one mole of a hydrogenated isophorone, are useful for making polycarbonate polymers with high glass transition temperatures and high heat distortion temperatures. Cyclohexyl bisphenol containing polycarbonates, or a combination comprising at least one of the foregoing with other bisphenol polycarbonates, are supplied by Bayer Co. under the APEC® trade name.

In further aspects, additional useful dihydroxy compounds are those compounds having the formula HO-$R^1$-OH include aromatic dihydroxy compounds of formula (4):

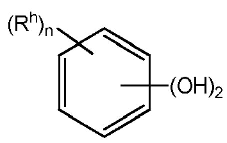

(5)

wherein each $R^h$ is independently a halogen atom, a $C_{1-10}$ hydrocarbyl such as a $C_{1-10}$ alkyl group, a halogen substituted $C_{1-10}$ hydrocarbyl such as a halogen-substituted $C_{1-10}$ alkyl group, and n is 0 to 4. The halogen is usually bromine.

In a further aspect, the branching agents include polyfunctional organic compounds containing at least three functional groups selected from hydroxyl, carboxyl, carboxylic anhydride, haloformyl, and mixtures thereof. Specific examples include trimellitic acid, trimellitic anhydride, trimellitic trichloride, tris-p-hydroxy phenyl ethane, isatin-bis-phenol,

tris-phenol TC (1,3,5-tris((p-hydroxyphenyl)isopropyl)benzene), tris-phenol PA (4(4(1,1-bis(p-hydroxyphenyl)-ethyl)alpha, alpha-dimethyl benzyl)phenol), 4-chloroformyl phthalic anhydride, trimesic acid, and benzophenone tetracarboxylic acid. The branching agents may be added at a level of from 0.05-2.0 weight percent. Branching agents and procedures for making branched polycarbonates are described in U.S. Pat. Nos. 3,635,895 and 4,001,184. All types of polycarbonate end groups are contemplated as being useful in the thermoplastic composition.

In an aspect, the melt polymerization process disclosed herein may include a carbonate source. As an example, a diaryl carbonate may be used as the carbonate source in melt polymerization processes. Exemplary diaryl carbonates that may be used according to the present disclosure are disclosed in U.S. Patent Nos. 7,365,149, 7547,799, 7,619,053, and 7,671,165. Of the diaryl carbonates disclosed in the patents, non-ester -substituted diaryl carbonates that may be used may include for example diphenyl carbonate, ditolylcarbonate, bis(chlorophenyl) carbonate, m-cresyl carbonate, and dinapthyl carbonate. Some specific and non-limiting examples of non-activated carbonates are bis(o-methylphenyl) carbonate, bis(p-cumylphenyl)carbonate, bis(p-(1,1,3,3-tetramethyl)butylphenyl)carbonate and bis(o-cyanophenyl)carbonate. Unsymmetrical combinations of these structures may also be used as non-activated carbonates.

A melt polymerization process may employ an activated carbonate. As used herein, the term "activated carbonate," is defined as a diarylcarbonate that is more reactive than diphenylcarbonate in transesterification reactions. Activated, or ester-substituted diaryl carbonates may increase transesterification reaction rates allowing the melt polymerization reaction to occur in few pieces of equipment, at reduced temperature, and/or in minimal residence times. Specific non-limiting examples of activated carbonates include bis(o-methoxycarbonylphenyl)carbonate, bis(o-chlorophenyl)carbonate, bis(o-nitrophenyl)carbonate, bis(o-acetylphenyl)carbonate, bis(o-phenylketonephenyl)carbonate, bis(o-formylphenyl)carbonate. Examples of specific ester-substituted diarylcarbonates include, but are not limited to, bis(methylsalicyl)carbonate (CAS Registry No. 82091-12-1) (also known as BMSC or bis(o-methoxycarbonylphenyl) carbonate), bis(ethylsalicyl)carbonate, bis(propylsalicyl)carbonate, bis(butylsalicyl)carbonate, bis(benzylsalicyl)carbonate, bis(methyl-4-chlorosalicyl)carbonate and the like. In one aspect, bis(methylsalicyl)carbonate is used as the activated carbonate in melt polycarbonate synthesis due to its lower molecular weight and higher vapor pressure. Some non-limiting examples of non-activating groups which, when present in an ortho position, would not be expected to result in activated carbonates are alkyl, cycloalkyl or cyano groups.

According to aspects of the present disclosure, the melt polycarbonate resin may be derived from a diarylcarbonate source. As a specific example, the melt polycarbonate resin may be derived from diphenyl carbonate.

In one aspect, an end-capping agent (also referred to as a chain-stopper) may optionally be used to limit molecular weight growth rate, and so control molecular weight in the polycarbonate. Exemplary chain-stoppers include certain monophenolic compounds (i.e., phenyl compounds having a single free hydroxy group), monocarboxylic acid chlorides, and/or monochloroformates. Phenolic chain-stoppers are exemplified by phenol and $C_1$-$C_{22}$ alkyl-substituted phenols such as p-cumyl-phenol, resorcinol monobenzoate, and p- and tertiary-butyl phenol, cresol, and monoethers of diphenols, such as p-methoxyphenol. Alkyl-substituted phenols with branched chain alkyl substituents having 8 to 9 carbon atoms may be specifically mentioned. Certain monophenolic UV absorbers may also be used as a capping agent, for example 4-substituted-2-hydroxybenzophenones and their derivatives, aryl salicylates, monoesters of diphenols such as resorcinol monobenzoate, 2-(2-hydroxyaryl)-benzotriazoles and their derivatives, 2-(2-hydroxyaryl)-1,3,5-triazines and their derivatives, and the like.

In another aspect, end groups may be derived from the carbonyl source (i.e., the diphenyl carbonate), from selection of monomer ratios, incomplete polymerization, chain scission, and the like, as well as any added end-capping groups, and may include derivatizable functional groups such as hydroxy groups, carboxylic acid groups, or the like. In one aspect, the endgroup of a polycarbonate, including a polycarbonate resin as defined herein, may comprise a structural unit derived from a diaryl carbonate, for example the diphenyl carbonate, where the structural unit may be an endgroup. In a further aspect, the endgroup is derived from an activated carbonate. Such endgroups may be derived from the transesterification reaction of the alkyl ester of an appropriately substituted activated carbonate, with a hydroxy group at the end of a polycarbonate polymer chain, under conditions in which the hydroxy group reacts with the ester carbonyl from the activated carbonate, instead of with the carbonate carbonyl of the activated carbonate. In this way, structural units derived from ester containing compounds or substructures derived from the activated carbonate and present in the melt polymerization reaction may form ester endgroups.

In various aspects of the present disclosure, the melt polymerization polycarbonate composition may have a Fries content of less than about 1500 ppm, less than about 1000ppm or less than about 800 ppm. Endpoints below 1500 ppm are included herein. Polycarbonates prepared according to a melt polymerization process or activated carbonate melt process such as those presented in US Patent Nos. 5,151,491 and 5,142,018 typically contain a significant concentration of Fries product when compared to an interfacial polycarbonate polymerization product. Although, a low level of Fries product may be tolerated in the melt process polycarbonate product, the presence of higher levels of Fries product may negatively impact performance characteristics of the polycarbonate, such as moldability and impact strength. With appropriate adjustments, the melt polymerization process may be performed to achieve a resultant polycarbonate composition with a particular Fries concentration.

The Fries product, or Fries rearrangement, arises as a side reaction occurring during the melt polymerization polycarbonate process. The term "Fries product" is defined as a structural unit of the product polycarbonate which upon hydrolysis of the product polycarbonate affords a carboxy- substituted dihydroxy aromatic compound bearing a carboxy group adjacent to one or both of the hydroxy groups of said carboxy-substituted dihydroxy aromatic compound. The resultant "Fries product" may serve as a site for branching of the polycarbonate chains thereby affecting flow and other properties of the polycarbonate. During preparation of the polycarbonate, the Fries rearrangement denotes the presence of a repeating unit in a polycarbonate having the following formula (7):

(6)

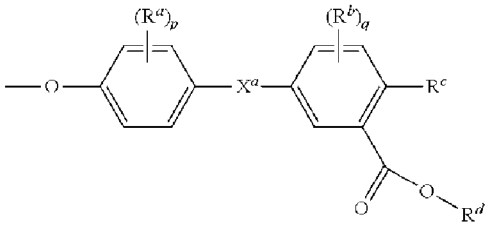

(7)

wherein $R^a$, $R^b$, p, q, and $X^a$ are defined as above. $R^c$ may be a hydroxyl group or a carbonate or ether. A polymer chain may form via the carbonate or ether group. The $R^d$ may be hydrogen or a substituted aryl group. A polymer chain may form via the substituted aryl group. For example, the following rearrangements (linear Fries, branched/ether Fries, and acid Fries) may occur:

Linear Fries

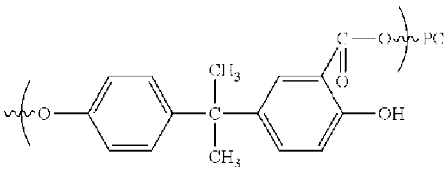

Branched/Ether Fries

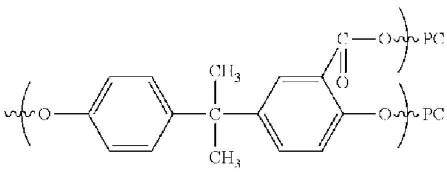

Acid Fries

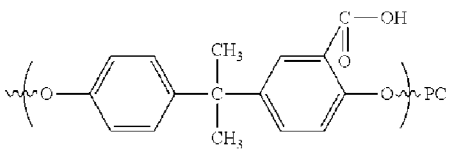

The total amount of branched Fries rearrangement may be adjusted during melt polymerization by modifying the temperatures and/or reaction times. Moreover, melt polymerization reagents may also be changed. For example, alkali metal hydroxides, such as sodium hydroxide, are employed as catalysts in the preparation of polycarbonate using the melt process. Alkali metal hydroxides, although effective catalysts in terms of rates of conversion of starting materials to product polycarbonate, tend to produce relatively high levels of Fries rearrangement product. This may occur because by-products formed at high temperature include Fries rearrangement of carbonate units along the growing polymer chains.

In various aspects of the present disclosure the Fries rearrangement of carbonate units along the growing polymer chains may also be measured to ensure that the process adjustments provide the desired amount of Fries rearrangement. The content of the various Fries components in polycarbonates may be determined by nuclear magnetic resonance (NMR) analysis. NMR peaks corresponding to branched Fries structure, linear Fries structure, and acid Fries

structure may be integrated to obtain the total Fries content. Quantification of Fries rearrangement content and the polycarbonate aryl hydroxy end-group content may be obtained based on the integral of the proton 1H NMR signal of the Fries components to the integral of the eight polycarbonate protons, as specifically described in the examples. In further aspects, the Fries content may be measured by high performance liquid chromatography HPLC or other known methods such as potassium hydroxide (KOH) methanolysis of a resin and may be reported as parts per million (ppm).

In various aspects of the present disclosure, the melt polymerization process may be performed in a series of reactors within which operating conditions such as temperature and pressure may be controlled. Typically a melt polymerization reactor system comprises an oligomer forming section and polymer molecular weight building section. The types of equipment used in each these sections are not particularly limited and may include for example mixing devices, stirred or unstirred vessels or reactors, kneaders, extruders, compounders, heat exchangers, flash tanks, transfer pipes, and the like. Examples of melt polymerization reaction systems and operating conditions are also disclosed in US Patent Nos. 7,365,149, 7,547,799 , 7,619,053 , and 7,671,165 , discussed above.

According to the methods disclosed herein, one skilled in the art may be able to readily select acceptable operating conditions and specific reaction equipment for the reactor systems and methods herein described. For example standard operating temperatures of reactor equipment in a melt production facility may be 50 °C to 500 °C. The higher the temperature, the faster the polymerization reaction. However, one skilled in the art will understand that as temperature increases undesired reaction byproducts may be formed and incorporated within the product polycarbonate and reaction components may be degraded. In some embodiments the melt polymerization conditions sufficient to produce polycarbonate include temperatures of 100 °C to 400 °C (e.g. 125 °C to 350 °C, for example 150 °C to 325 °C).

In one aspect, control over the reactor system may allow for the removal of the phenolic byproduct from the reaction system. As the phenolic byproduct is removed, the melt transesterification reaction may be driven by equilibrium displacement. As the phenolic byproduct is removed the reaction may be driven toward building the molecular weight of the polycarbonate. The structure of the phenolic byproduct would depend upon the diaryl carbonate used as the carbonate source.

In a further aspect, volatile monohydric phenol may be removed from the molten reactants by distillation and the polymer is isolated as a molten residue. In another aspect, a useful melt process for making polycarbonates utilizes a diaryl carbonate ester having electron-withdrawing substituents on the aryls. Examples of specifically useful diaryl carbonate esters with electron withdrawing substituents include bis(4-nitrophenyl)carbonate, bis(2-chlorophenyl)carbonate, bis(4-chlorophenyl)carbonate, bis(methyl salicyl)carbonate, bis(4-methylcarboxylphenyl)carbonate, bis(2-acetylphenyl)carboxylate, bis(4-acetylphenyl)carboxylate, or a combination comprising at least one of the foregoing.

In one aspect, the reactants for the polymerization reaction using a diphenyl carbonate may be charged into a reactor either in the solid form or in the molten form. Initial charging of reactants into a reactor and subsequent mixing of these materials under reactive conditions for polymerization may be conducted in an inert gas atmosphere such as a nitrogen atmosphere. The charging of one or more reactants may also be done at a later stage of the polymerization reaction. Mixing of the reaction mixture may be accomplished by any methods known in the art, such as by stirring. Reactive conditions include time, temperature, pressure and other factors that affect polymerization of the reactants. Typically the activated aromatic carbonate such as diphenyl carbonate may be added at a mole ratio of 0.8 to 1.3, and more preferably 0.9 to 1.3, and all subranges there between, relative to the total moles of monomer unit compounds (i.e., aromatic dihydroxy compound, and aliphatic diacid or diol). In a specific aspect, the molar ratio of diphenyl carbonate to monomer unit compounds is 1.013 to 1.29, specifically 1.015 to 1.028.

In one aspect, the progress of the reaction may be monitored by measuring the melt viscosity or the weight average molecular weight of the reaction mixture using techniques known in the art such as gel permeation chromatography or other methods. These properties may be measured by taking discrete samples or may be measured on-line. After the desired melt viscosity and/or molecular weight is reached, the final polycarbonate product may be isolated from the reactor in a solid or molten form. It will be appreciated by a person skilled in the art, that the method of making polycarbonate as described in the preceding sections may be made in a batch or a continuous process and the process disclosed herein is preferably carried out in a solvent free mode which characterizes a melt polymerization process. Reactors chosen should ideally be self-cleaning and should minimize any "hot spots." However, vented extruders similar to those that are commercially available may be used.

One exemplary polycarbonate composition is shown below by formula (I):

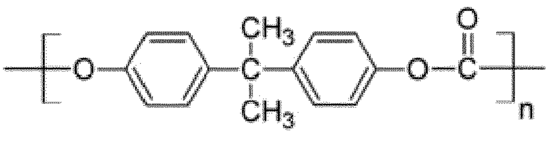
(I)

Polycarbonates are known to those of skill in the art. Polycarbonates, including aromatic carbonate chain units,

include compositions having structural units of the formula (II):

(II)

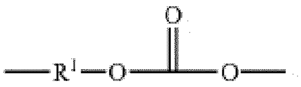

in which the $R^1$ groups are aromatic, aliphatic or alicyclic radicals. Preferably, $R^1$ is an aromatic organic radical, e.g., a radical of the formula (III):

$-A^1-Y^1-A^2-$ (III)

wherein each of $A_1$ and $A_2$ is a monocyclic divalent aryl radical and Y1 is a bridging radical having zero, one, or two atoms which separate A1 from A2. In an exemplary embodiment, one or more atoms separate A1 from A2. Illustrative examples of radicals of this type are --O--,S--, --S(O)--, --$S(O_2)$--, --C(O)--, methylene, cyclohexyl-methylene, 2-[2,2,1]-bicycloheptylidene, ethylidene, isopropylidene, neopentylidene, cyclohexylidene, cyclopentadecylidene, cyclododecylidene, adamantylidene, or the like. In another embodiment, zero atoms separate A1 from A2, with an illustrative example being bisphenol. The bridging radical Y1 can be a hydrocarbon group or a saturated hydrocarbon group such as methylene, cyclohexylidene or isopropylidene.

Polycarbonates can be produced by, e.g., melt processes and also by interfacial reaction polymer processes, both of which are well known in the art. An interfacial process may use precursors such as dihydroxy compounds in which only one atom separates $A^1$ and $A^2$. As used herein, the term "dihydroxy compound" includes, for example, bisphenol compounds having general formula (IV) as follows:

(IV)

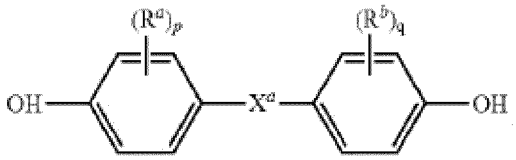

wherein $R^a$ and $R^b$ each independently represent hydrogen, a halogen atom, or a monovalent hydrocarbon group; p and q are each independently integers from 0 to 4; and $X^a$ represents one of the groups of formula (V):

(V)

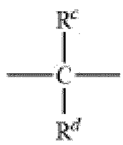

or

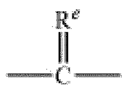

wherein $R^e$ and $R^d$ each independently represent a hydrogen atom or a monovalent linear or cyclic hydrocarbon group, and $R^e$ is a divalent hydrocarbon group.

Examples of the types of bisphenol compounds that can be represented by formula (IV) include the bis(hydroxyaryl)alkane series. Other bisphenol compounds that can be represented by formula (IV) include those where X is --O--, --S--, --SO-- or -SO22--.Other bisphenol compounds that can be utilized in the polycondensation of polycarbonate are represented by the formula (VI)

(VI)

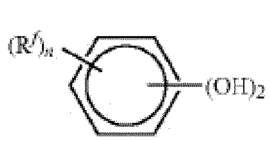

;

wherein, $R^f$, is a halogen atom of a hydrocarbon group having 1 to 10 carbon atoms or a halogen substituted hydrocarbon group; n is a value from 0 to 4. When n is at least 2, $R^f$ can be the same or different. Examples of bisphenol compounds represented by formula (V), are resorcinol, substituted resorcinol compounds such as 3-methyl resorcin, and the like.

Bisphenol compounds (e.g., bisphenol A), such as 2,2,2',2'-tetrahydro-3,3,3',3'-tetramethyl-1,1'-spirobi-[IH-indene]-6,6'-- diol represented by the following formula (VII) can also be used.

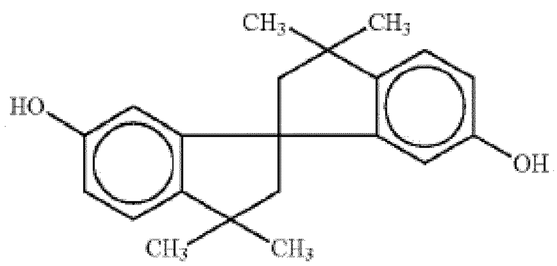

(VII)

Branched polycarbonates, as well as blends of linear polycarbonate and a branched polycarbonate can also be used. Branched polycarbonates can be prepared by adding a branching agent during polymerization. These branching agents can include polyfunctional organic compounds containing at least three functional groups, which can be hydroxyl, carboxyl, carboxylic anhydride, haloformyl, and combinations including at least one of the foregoing branching agents. Specific examples include trimellitic acid, trimellitic anhydride, trimellitic trichloride, tris-p-hydroxy phenyl ethane, isatin-bis-phenol, tris-phenol TC (1,3,5-tris((p-hydroxyphenyl)isopropyl)benzene), tris-phenol PA (4(4(1,1-bis(p-hydroxyphenyl)-ethyl)alpha,alpha-dimethyl benzyl)phenol), 4-chloroformyl phthalic anhydride, trimesic acid, benzophenone tetracarboxylic acid, or the like, or combinations including at least one of the foregoing branching agents. The branching agents can be added at a level of about 0.05 to about 2.0 weight percent (wt %), based upon the total weight of the polycarbonate in a given layer.

In some aspects of the disclosure, the polycarbonate resin has an endcap level of at least about 95%. Polycarbonate resins having this endcap level may generally be produced by an interfacial polymerization process. Purely by way of example, in one particular interfacial polymerization process in which the polycarbonate is BPA polycarbonate, the BPA polycarbonate is produced by amine catalyzed interfacial polycondensation of bisphenol A and phosgene. In contrast to other known methods for forming polycarbonates (such as melt transesterification processes) in which the polycarbonate has an endcap level of less than about 95%, polycarbonates formed by an interfacial polymerization process are characterized as having an endcap level of at least about 95%. In some aspects, the polycarbonate resin has an endcap level of at least about 98%, or even an endcap level of at least about 99%. In further aspects, the polycarbonate resin is substantially, fully endcapped, that is, endcapping is about 100 .%.

The end cap level (EC%) may be determined by the measurement of the OH groups and the molecular weight concentration of the polycarbonate resin. For example, the ratio of the OH group content (BPA groups for the melt PC) referred to the total end groups (BPA + PhOH (phenol) from DPC groups) of the polycarbonate composition provides the EC%. A higher EC% value may indicate a lower concentration of OH endgroups.

In the present disclosure, an improvement in the impact properties of polycarbonate melt resin for low viscosity grades with melt volume rate (MVR) from about 20 $cm^3$/min (1.2 Kg - 300°C ISO 1133) to about 30 $cm^3$/min is shown. This improvement has been achieved by blending the melt PC resin with other PC interfacial resin. The interfacial PC resin component has a different type of endcap compared with traditional endcaps groups of the melt PC resin. The endcap groups of the interfacial PC resin are based on the use of phenol and other bulkier molecules as p-cumyl phenol and p-terbutyl phenol compare with the typical endgroups (BPA and phenol) present in the melt resin.

The increasing of the bulky endgroups concentration (by adding different proportions of one PC interfacial grade based on 100 % p-cumyl phenol end cap to a melt PC resin) may improve the impact performance of the melt material compared with a substantially similar resin in the absence of the interfacial polycarbonate.

In addition to the foregoing components, the polycarbonate compositions from which the disclosed articles are formed may comprise a balance amount of one or more additive materials ordinarily incorporated in polycarbonate resin compositions of this type, with the proviso that the additives are selected so as to not significantly adversely affect the desired properties of the polycarbonate composition. The melt polycarbonate resin may comprise one or more suitable additives with the proviso that the additives are selected so as to not significantly adversely affect the desired properties such as transparency, impact strength, heat stability, and/or weathering resistance of the melt polycarbonate. The reaction mixture may optionally be blended with any conventional additives used in thermoplastics applications, such as preparing molded articles. These additives include, without limitation, quenchers, UV stabilizers/absorbers, antioxidants, heat stabilizers, mold release agents, coloring agents, antistatic agents, slip agents, anti-blocking agents, lubricants, anti-clouding agents, coloring agents, natural oils, synthetic oils, waxes, organic fillers, inorganic fillers, branching agents and mixtures thereof. Combinations of additives can be used. Such additives may be mixed at a suitable time during the mixing of the components for forming the composition.

In one aspect, a blend of the melt polycarbonate resin composition and additives may be formed which aids in processing the blend to form the desired molded article, such as an optical article (disk or lens), automobile lamp components or the like. The blend may optionally contain about 0.0001 to about 10 percent by weight of the desired additives. In an aspect, the blend contains about 0.0001 to about 1.0% by weight of the desired additives.

The composition may further include an anti-static additive. An exemplary anti-static additive may comprise a halogenated carbon sulfonic acid salt of a polysubstituted phosphonium compound. In one aspect, the composition may include tetrabutyl phosphonium per fluoro-butylsulfonate.

Exemplary ultraviolet (UV) absorbers or UV protection agents may include, but are not limited to, salicylic acid UV absorbers, benzophenone UV absorbers, benzotriazole UV absorbers, cyanoacrylate UV absorbers and mixtures thereof. An exemplary UV protection agent/absorber may include 2-(2 hydroxy-5-t-octylphenyl) benzotriazole or phenol, 2,2'-methylene-bis(6-(2H-benzotriazol-2-yl)-4-(1,1,3,3-tetramethylbutyl)). Examples of heat-resistant stabilizers, include, but are not limited to, phenol stabilizers, organic thioether stabilizers, organic phosphite stabilizers, hindered amine stabilizers, epoxy stabilizers and mixtures thereof. The heat-resistant stabilizer may be added in the form of a solid or liquid. Examples of the mold-release agents include, but are not limited to natural and synthetic paraffins, polyethylene waxes, fluorocarbons, and other hydrocarbon mold-release agents; stearic acid, hydroxystearic acid, and other higher fatty acids, hydroxyfatty acids, and other fatty acid mold-release agents; stearic acid amide, ethylenebis(stearamide), and other fatty acid amides, alkylene bisfatty acid amides, and other fatty acid amide mold-release agents; stearyl alcohol, cetyl alcohol, and other aliphatic alcohols, polyhydric alcohols, polyglycols, polyglycerols and other alcoholic mold release agents; butyl stearate, pentaerythritol tetrastearate, and other lower alcohol esters of fatty acids, polyhydric alcohol esters of fatty acids, polyglycol esters of fatty acids, and other fatty acid ester mold release agents; silicone oil and other silicone mold release agents, and mixtures of any of the aforementioned. The coloring agent may be either pigments or dyes. Inorganic coloring agents and organic coloring agents may be used separately or in combination. Examples of branching agents include, without limitation, THPE (phenol, 4,4',4" -ethylidynetris), 9-carboxyoctadecandioic acid, or 1,3,5-trihydroxybenzene. Additives such as plasticizers, lubricants, and/or mold release agents additive are generally used in amounts of about 0.01 weight percent (wt. %) to about 20 wt. %, optionally about 0.5 wt. % to about 10 wt. % the polycarbonate blend composition. In one aspect, the mold release agent is glycerol tristearate

In a further aspect, the disclosed blended thermoplastic compositions may further comprise a primary antioxidant or "stabilizer" (e.g., a hindered phenol) and, optionally, a secondary antioxidant (e.g., a phosphate and/or thioester). In one aspect, the antioxidant is a primary antioxidant, a secondary antioxidant, or combinations thereof. In a still further aspect, the primary antioxidant is selected from a hindered phenol and secondary aryl amine, or a combination thereof. An exemplary antioxidant may include octadecyl 3-(3,5-di-tert-butyl-4-hydroxyphenyl)propionate as a sterically hindered phenolic antioxidant. Antioxidants may generally be used in amounts of about 0.01 wt. % to about 3 wt. %, optionally about 0.05 wt. % to about 2.0 wt. % of the blended thermoplastic composition. In some aspects, hydrolytic and thermal stabilizers may be used with the melt polycarbonate resin. Thermal stabilizers are generally used in amounts of about 0.01 wt. % to about 5 wt. %, optionally about 0.05 wt. % to about 2.0 wt. % of the polycarbonate blend composition.

In a further aspect, anti-drip agents may also be present. In a further aspect, the anti-drip agent is a styrene-acrylonitrile copolymer encapsulated polytetrafluoroethylene. Exemplary anti-drip agents may include a fibril forming or non-fibril forming fluoropolymer such as polytetrafluoroethylene (PTFE) or PTFE encapsulated in SAN known as TSAN. The anti-drip agent may be present in an amount from about 0.01 wt. % to about 3 wt. %.

## ARTICLES OF MANUFACTURE

The melt polycarbonate resin with the interfacial polycarbonate component as disclosed herein may be useful as an alternative to polycarbonate resins prepared according to only an interfacial polymerization process. In yet further aspects, the properties of the melt polycarbonate resin as disclosed herein may be particularly useful in applications where articles formed therefrom are subjected to prolonged weathering, wear, or use. Accordingly, the melt polycarbonate resin with the interfacial polycarbonate component may be used to form articles typically formed from interfacial polymerization polycarbonate resins. The transparency, flow, and impact performance of the articles may be comparable to the qualities achieved according to interfacial polymerization process.

As such, the disclosed melt polycarbonate resin with the interfacial polycarbonate component may be used to manufacture articles for use in electronic, automotive, imaging, healthcare, or optical devices. Devices and applications may include: anti-fog windows; lenses and/or transparent covers for lighting applications such as automotive lighting, street lighting, outdoor lighting, and high efficiency lighting such as light emitting diode LED applications, organic LED applications, black light frames for LED/LCD displays, black light holders for LED/LCD displays, LCD displays, fluorescent lighting applications, vapor gas discharge lighting application, and neon light application, which may produce less heat as a byproduct, compared to conventional light sources; optical lenses including camera and viewing lenses, e.g., for mobile telephone cameras, and for digital still photography cameras, mirrors, telescopic lenses, binoculars, automotive camera lenses, and ophthalmic items such as eyewear including sunglasses, sunglass lenses, protective goggles, sports eyewear, face shields, and prescription lenses. Electro-optical devices may also include cathode ray tubes, fluorescent lighting, vapor gas discharge light sources, and neon light, as well as light emitting diodes, organic light emitting diodes, plasma, and liquid crystal screens.

Articles formed from the compositions and methods of the present disclosure may be particularly useful for the

following applications: mobile phones, mobile computing devices, cameras, video recorders, projectors, corrective lenses, diffusers, or copiers. In yet further examples, the polycarbonate resins may be useful to form articles for use in devices such as lenses for use in portable electronics applications including cell phones, cameras, personal digital assistants, DVD players and recording devices, and the like. Furthermore, articles and products made from the disclosed compositions may be also be used in a variety of applications including thin-wall articles, where transparency, precision as defined by a high degree of reproducibility, retention of mechanical properties including impact strength, and precise optical properties are required. In a yet further example, the optically transparent, melt polycarbonate articles may be weatherable, or resistant to outdoor weathering conditions of higher heat and full sun conditions. The articles may be used to protect optoelectronic devices, such as solar cells, situated in outdoor working environments for extended periods of time while maintaining impact strength.

In one aspect, the present disclosure pertains to shaped, formed, or molded articles comprising polycarbonate compositions prepared according to the melt polymerization process disclosed herein. The polycarbonate compositions may be molded into useful shaped articles by a variety of means such as injection molding, extrusion, rotational molding, blow molding and thermoforming to form articles. The polycarbonate compositions described herein may also be made into film and sheet as well as components of laminate systems. The articles comprising the disclosed polycarbonate compositions may be, but are not limited to, computer and business machine housings such as housings for high end laptop personal computers, monitors, hand held electronic device housings such as housings for smart phones, tablets, LCD displays, compact and video discs, music devices electrical, connectors, and components of lighting fixtures, ornaments, home appliances, and the like.

In a further aspect, the molded articles may be used to manufacture devices in the healthcare field. In still a further aspect, non-limiting examples of such devices in the healthcare field which may use the disclosed blended thermoplastic compositions include drug delivery devices, syringes, stopcocks, lures, renal or blood care devices, blood filters, dialysis housings, meters for dialysis housings, pumps for dialysis housings, insulin pens, or face masks.

In a further aspect, the molded articles may be used to manufacture devices in the automotive field. In a still further aspect, non-limiting examples of such devices in the automotive field which may use the disclosed blended thermoplastic compositions in the vehicle's interior include adaptive cruise control, headlight sensors, automotive bezels, automotive reflectors, windshield wiper sensors, and door/window switches. In still a further aspect, the disclosed blended thermoplastic composition may be used on the vehicle's exterior. Various combinations of elements of this disclosure are encompassed by this disclosure, *e.g.* combinations of elements from dependent claims that depend upon the same independent claim.

## METHODS OF MANUFACTURE

In various aspects of the present disclosure, the melt polymerization reaction may be carried out in typical polymerization equipment, such as one or more continuously stirred reactors (CSTRs), plug flow reactors, wire wetting fall polymerizers, free fall polymerizers, wiped film polymerizers, BANBURY® mixers, single or twin screw extruders, or combinations of the foregoing. In one aspect, volatile monohydric phenol generated in situ may be removed from the molten reactants by distillation and the polymer is isolated as a molten residue.

The melt polycarbonate resin compositions of the present disclosure may be blended with the aforementioned ingredients, including with the interfacial polycarbonate component, by a variety of methods involving intimate admixing of the materials with any additional additives desired in the formulation. Because of the availability of melt blending equipment in commercial polymer processing facilities, melt processing methods are generally preferred. Illustrative examples of equipment used in such melt processing methods include: co-rotating and counter-rotating extruders, single screw extruders, co-kneaders, disc-pack processors and various other types of extrusion equipment. The temperature of the melt in the present process is preferably minimized in order to avoid excessive degradation of the resins. It is often desirable to maintain the melt temperature between about 230 °C and about 350 °C in the molten resin composition, although higher temperatures can be used provided that the residence time of the resin in the processing equipment is kept short. In some embodiments the melt processed composition exits processing equipment such as an extruder through small exit holes in a die. The resulting strands of molten resin are cooled by passing the strands through a water bath. The cooled strands may be chopped into small pellets for packaging and further handling.

Compositions may be manufactured by various methods, including batch or continuous techniques that employ kneaders, extruders, mixers, and the like. For example, the composition may be formed as a melt blend employing a twin-screw extruder. In some embodiments at least some of the components are added sequentially. Alternatively, the sequential addition of the components may be accomplished through multiple extrusions. A composition may be made by pre-extrusion of selected components. A second extrusion may then be employed to combine the pre-extruded components with the remaining components.

As described herein, the present disclosure relates to a method of making a polycarbonate composition from a melt polycarbonate polymerization process. The composition of the present disclosure may be formed using any known

method of combining multiple components to form a polymer resin. In one aspect, the components are first blended in a high-speed mixer. Other low shear processes including but not limited to hand mixing may also accomplish this blending. The blend is then fed into the throat of a twin-screw extruder via a hopper. Alternatively, one or more of the components may be incorporated into the composition by feeding directly into the extruder at the throat and/or downstream through a sidestuffer. The extruder is generally operated at a temperature higher than that necessary to cause the composition to flow. The extrudate is immediately cooled in a water batch and pelletized. The pellets so prepared when cutting the extrudate may be one-fourth inch long or less as desired. Such pellets may be used for subsequent molding, shaping, or forming. In one aspect, the blend composition is formed by extrusion blending.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; and the number or type of aspects described in the specification.

## DEFINITIONS

It is to be understood that the terminology used herein is for the purpose of describing particular aspects only and is not intended to be limiting. As used in the specification and in the claims, the term "comprising" may include the aspects "consisting of" and "consisting essentially of." Unless defined otherwise, all technical and scientific terms used herein have the same meaning as commonly understood by one of ordinary skill in the art to which this disclosure belongs. In this specification and in the claims which follow, reference will be made to a number of terms which shall be defined herein. All publications mentioned herein are incorporated herein by reference to disclose and describe the methods and/or materials in connection with which the publications are cited.

As used in the specification and the appended claims, the singular forms "a," "an" and "the" include plural referents unless the context clearly dictates otherwise. Thus, for example, reference to "a polycarbonate" includes mixtures of two or more such polycarbonates. Furthermore, for example, reference to a filler includes mixtures of two or more such fillers.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" mean that the subsequently described event, condition, component, or circumstance may or may not occur, and that the description includes instances where said event or circumstance occurs and instances where it does not.

As used herein, the term or phrase "effective," "effective amount," or "conditions effective to" refers to such amount or condition that is capable of performing the function or property for which an effective amount is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation.

Disclosed are component materials to be used to prepare disclosed compositions of the disclosure as well as the compositions themselves to be used within methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed.

Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight, of a particular element or component in a composition or article denotes the weight relationship between the element or component and any other elements or components in the composition or article for which a part by weight is expressed. Thus, in a composition containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included. For example if a particular element or component in a composition or article is said to have 8% weight, it is understood that this percentage is relation to a total compositional percentage of 100%.

Compounds disclosed herein are described using standard nomenclature. For example, any position not substituted by any indicated group is understood to have its valence filled by a bond as indicated, or a hydrogen atom. A dash ("-") that is not between two letters or symbols is used to indicate a point of attachment for a substituent. For example, -CHO is attached through carbon of the carbonyl group. Unless defined otherwise, technical and scientific terms used herein have the same meaning as is commonly understood by one of skill in the art to which this disclosure belongs.

The term "comparable" as used herein may refer to similarity between given resin compositions described herein. Comparable may be used to express that properties, or the quantified values of given properties, are similar to or commensurate with the properties of another.

As used herein, the term "substantially identical reference composition" refers to a composition that is substantially identical to the inventive composition by consisting essentially of substantially the same proportions and components but in the absence of a single component.

The term "transparency" as used herein may refer to a level of transmittance for a resin composition that is greater than 50 %, including exemplary transmittance values of at least 60 %, 70 %, 80 %, 85 %, 90 %, and 95 %, or any range of transmittance values derived from the above exemplified values. In some examples, the resin composition may exhibit a transmittance value of greater than 85 %. Transmittance may be measured for a disclosed resin composition according to ASTM method D1003.

As used herein, the terms "number average molecular weight" or "Mn" can be used interchangeably, and refer to the statistical average molecular weight of all the polymer chains in the sample and is defined by the formula:

$$Mn = \frac{\sum N_i M_i}{\sum N_i},$$

where $M_i$ is the molecular weight of a chain and $N_i$ is the number of chains of that molecular weight. Mn can be determined for polymers, such as polycarbonate polymers or polycarbonate-PMMA copolymers, by methods well known to a person having ordinary skill in the art. It is to be understood that as used herein, Mn is measured gel permeation chromatography and as calibrated with polycarbonate standards. For example, gel permeation chromatography can be carried out using a crosslinked styrene-divinyl benzene column, at a sample concentration of 1 milligram per milliliter with appropriate mobile phase solvents.

As used herein, the terms "weight average molecular weight" or "Mw" can be used interchangeably, and are defined by the formula:

$$Mw = \frac{\sum N_i {M_i}^2}{\sum N_i M_i},$$

where $M_i$ is the molecular weight of a chain and $N_i$ is the number of chains of that molecular weight. Compared to Mn, Mw takes into account the molecular weight of a given chain in determining contributions to the molecular weight average. Thus, the greater the molecular weight of a given chain, the more the chain contributes to the Mw. It is to be understood that as used herein, Mw is measured gel permeation chromatography. In some cases, Mw is measured by gel permeation chromatography and calibrated with polycarbonate standards. In further aspects, Mw is measured by gel permeation chromatography and calibrated with polystyrene standards. Gel permeation chromatography can be carried out using a crosslinked styrene-divinyl benzene column, at a sample concentration of about 1 milligram per milliliter with appropriate mobile phase solvents.

**Aspects:**

In various aspects, the present disclosure pertains to and includes at least the following aspects.

Aspect 1. An article formed from a composition comprising: a melt polycarbonate resin derived from diphenyl carbonate; and an interfacial polycarbonate resin mixed with the melt polycarbonate resin, wherein the composition exhibits a melt volume rate of between about 20 $cm^3$/10 minutes and about 30 $cm^3$/10 minutes at 1.2 kg and 300 °C, wherein the article formed from the composition exhibits an IZOD Notched Impact performance that is greater than an IZOD Notched Impact performance of an article formed from a comparator composition consisting essentially of the melt polycarbonate resin within the ductile to brittle transition temperature range of the composition and the comparator composition.

Aspect 2. An article formed from a composition comprising: a melt polycarbonate resin derived from diphenyl carbonate; and an interfacial polycarbonate resin mixed with the melt polycarbonate resin, wherein the composition exhibits a melt volume rate of between about 20 $cm^3$/10 minutes and about 30 $cm^3$/10 minutes at 1.2 kg and 300 °C, wherein the article formed from the composition exhibits an IZOD Notched Impact performance that is greater than an IZOD Notched Impact performance of an article formed from a comparator composition consisting essentially of the melt polycarbonate resin at a temperature in the range of about -20 °C and about 0 °C.

Aspect 3. The article of any one of aspects 1-2, wherein the article is transparent.

Aspect 4. The article of any one of aspects 1-3, wherein the interfacial polycarbonate resin has an endcap of phenol or any bulkier groups as p-cumylphenol, p-tertbutylphenol or combination thereof

Aspect 5. The article of any one of aspects 1-4, wherein the composition exhibits a melt volume rate of between about 21 $cm^3$/10 minutes and about 26 $cm^3$/10 minutes at 1.2kg and 300 °C.

Aspect 6. The article of any one of aspects 1-4, wherein the composition exhibits a melt volume rate of 26 $cm^3$/10 minutes at 1.2 kg and 300 °C.

Aspect 7. The article of any one of aspects 1-6, wherein the article formed from the composition exhibits an IZOD Notched Impact performance that is greater than an IZOD Notched Impact performance of an article formed from a comparator composition consisting essentially of the melt polycarbonate resin at about -5 °C.

Aspect 8. The article of any one of aspects 1-6, wherein the article formed from the composition exhibits an IZOD Notched Impact performance that is greater than an IZOD Notched Impact performance of an article formed from a comparator composition consisting essentially of the melt polycarbonate resin at a temperature between about -15 °C and about 0 °C.

Aspect 9. The article of any one of aspects 1-6, wherein the article formed from the composition exhibits an IZOD Notched Impact performance that is greater than an IZOD Notched Impact performance of an article formed from a comparator composition consisting essentially of the melt polycarbonate resin at a temperature between about -10 °C and about -5 °C.

Aspect 10. The article of any one of aspects 1-9, wherein the composition comprises about 20 wt% to about 80% of the melt polycarbonate resin relative to 100 wt% of all polycarbonate resin in the composition.

Aspect 11. The article of any one of aspects 1-9, wherein the composition comprises about 20 wt% of the melt polycarbonate resin and about 80% of the interfacial polycarbonate resin relative to 100 wt% of all polycarbonate resin in the composition.

Aspect 12. The article of any one of aspects 1-9, wherein the composition comprises about 40 wt% of the melt polycarbonate resin and about 60% of the interfacial polycarbonate resin relative to 100 wt% of all polycarbonate resin in the composition.

Aspect 13. The article of any one of aspects 1-9, wherein the composition comprises about 60 wt% of the melt polycarbonate resin and about 40% of the interfacial polycarbonate resin relative to 100 wt% of all polycarbonate resin in the composition.

Aspect 14. The article of any one of aspects 1-9, wherein the composition comprises about 80 wt% of the melt polycarbonate resin and about 20% of the interfacial polycarbonate resin relative to 100 wt% of all polycarbonate resin in the composition.

Aspect 15. The article of any one of aspects 1-14, wherein the article is used in automotive applications.

Aspect 16. The article of aspect 15, wherein the article comprises automotive bezels, automotive reflectors, an automotive interior trim, or an automotive exterior.

Aspect 17. The article of any one of aspects 1-14, wherein the article is used in electrical and lighting applications.

Aspect 18. The article of aspect 17, wherein the article comprises electrical components, electrical enclosures including MCCD, black light frames for LED/LCD displays, black light holders for LED/LCD displays, lighting fixtures, or lighting covers.

Aspect 19. The article of any one of aspects 1-14, wherein the article is used in healthcare applications.

Aspect 20. The article of aspect 19, wherein the article comprises a drug delivery device.

Aspect 21. The article of aspect 20, wherein the article comprises syringes, stopcocks, or lures.

Aspect 22. The article of aspect 19, wherein the article comprises renal or blood care devices.

Aspect 23. The article of aspect 22, wherein the article comprises blood filters, dialysis housings, meters for dialysis housings, pumps for dialysis housings, or insulin pens.

Aspect 24. The article of aspect 19, wherein the article comprises a face mask.

Aspect 25. The article of any one of aspects 1-14, wherein the article is used in electrical appliance application.

Aspect 26. The article of aspect 25, wherein the article comprises housings for electrical components of consumer electronics.

Aspect 27. The article of aspect 26, wherein the article comprises a housing for electrical components of personal computers, laptop computers, personal tablets, mobile phones, LCD displays, compact discs, or video discs.

Aspect 28. The article of any one of aspects 1-14, wherein the article is used in eyewear applications.

Aspect 29. The article of aspect 28, wherein the article comprises sunglass lenses, sports eyewear, or safety glasses.

Aspect 30. A method of forming the article of any one of aspects 1-14.

Aspect 31. A composition comprising: a melt polycarbonate resin derived from diphenyl carbonate; and an interfacial polycarbonate resin mixed with the melt polycarbonate resin, wherein the composition exhibits a melt volume rate of between about 20 $cm^3$/10 minutes and about 30 $cm^3$/10 minutes at 1.2 kg and 300 °C, wherein an article formed from the composition exhibits an IZOD Notched Impact performance that is greater than an IZOD Notched Impact performance of an article formed from a comparator composition consisting essentially of the melt polycarbonate resin within the ductile to brittle transition temperature range of the composition and the comparator composition.

Aspect 32. A composition comprising: a melt polycarbonate resin derived from diphenyl carbonate; and an interfacial polycarbonate resin mixed with the melt polycarbonate resin, wherein the composition exhibits a melt volume rate of between about 20 $cm^3$/10 minutes and about 30 $cm^3$/10 minutes at 1.2 kg and 300 °C, wherein an article formed from the composition exhibits an IZOD Notched Impact performance that is greater than an IZOD Notched Impact performance of an article formed from a comparator composition consisting essentially of the melt polycarbonate resin at a temperature in the range of about -20 °C and about 0 °C.

Aspect 33. The composition of any one of aspects 31-32, wherein the article formed from the composition is transparent.

Aspect 34. The composition of any one of aspects 31-33, wherein the interfacial polycarbonate resin has an endcap of p-cumyl phenol, p-tertbutyl phenol, phenol, phosgene, or a combination thereof.

Aspect 35. The composition of any one of aspects 31-34, wherein the composition exhibits a melt volume rate of between about 21 $cm^3$/10 minutes and about 26 $cm^3$/10 minutes at 1.2kg and 300 °C.

Aspect 36. The composition of any one of aspects 31-34, wherein the composition exhibits a melt volume rate of 26 $cm^3$/10 minutes at 1.2 kg and 300 °C.

Aspect 37. The composition of any one of aspects 31-36, wherein the article formed from the composition exhibits an IZOD Notched Impact performance that is greater than an IZOD Notched Impact performance of an article formed from a comparator composition consisting essentially of the melt polycarbonate resin at about -5 °C.

Aspect 38. The composition of any one of aspects 31-36, wherein the article formed from the composition exhibits an IZOD Notched Impact performance that is greater than an IZOD Notched Impact performance of an article formed from a comparator composition consisting essentially of the melt polycarbonate resin at a temperature between about -15 °C and about 0 °C.

Aspect 39. The composition of any one of aspects 31-36, wherein the article formed from the composition exhibits an IZOD Notched Impact performance that is greater than an IZOD Notched Impact performance of an article formed from a comparator composition consisting essentially of the melt polycarbonate resin at a temperature between about -10 °C and about -5 °C.

Aspect 40. The composition of any one of aspects 31-39, wherein the composition comprises about 20 wt% to about 80% of the melt polycarbonate resin relative to 100 wt% of all polycarbonate resin in the composition.

Aspect 41. The composition of any one of aspects 31-39, wherein the composition comprises about 20 wt% of the melt polycarbonate resin and about 80% of the interfacial polycarbonate resin relative to 100 wt% of all polycarbonate resin in the composition.

Aspect 42. The composition of any one of aspects 31-39, wherein the composition comprises about 40 wt% of the melt polycarbonate resin and about 60% of the interfacial polycarbonate resin relative to 100 wt% of all polycarbonate resin in the composition.

Aspect 43. The composition of any one of aspects 31-39, wherein the composition comprises about 60 wt% of the melt polycarbonate resin and about 40% of the interfacial polycarbonate resin relative to 100 wt% of all polycarbonate resin in the composition.

Aspect 44. The composition of any one of aspects 31-39, wherein the composition comprises about 80 wt% of the melt polycarbonate resin and about 20% of the interfacial polycarbonate resin relative to 100 wt% of all polycarbonate resin in the composition.

Aspect 45. A method of making the composition of any one of aspects 31-44.

Without further elaboration, it is believed that one skilled in the art can, using the description herein, utilize the present disclosure. The following examples are included to provide addition guidance to those skilled in the art of practicing the claimed disclosure. The examples provided are merely representative of the work and contribute to the teaching of the present disclosure. Accordingly, these examples are not intended to limit the disclosure in any manner.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon. Nothing herein is to be construed as an admission that the present disclosure is not entitled to antedate such publication by virtue of prior disclosure. Further, the dates of publication provided herein can be different from the actual publication dates, which can require independent confirmation.

## EXAMPLES

The following examples are put forth so as to provide those of ordinary skill in the art with a complete disclosure and description of how the methods, devices, and systems disclosed and claimed herein are made and evaluated, and are intended to be purely exemplary and are not intended to limit the disclosure. Efforts have been made to ensure accuracy with respect to numbers (*e.g.*, amounts, temperature, etc.), but some errors and deviations should be accounted for. Unless indicated otherwise, parts are parts by weight, temperature is in degrees Celsius (°C) or is at ambient temperature, and pressure is at or near atmospheric.

A battery of samples with the same viscosity (MVR = 26 $cm^3$/10 min - 1.2 Kg) at different proportions of melt and interfacial resins was generated by an off line blending step in an extruder. The term "off line" is defined as a non-continuous step in a blending process as opposed to an on line blending of additives in the extruder in a continuous process. The samples were generated by adjusting the polymers proportions, as illustrated in **Table 1:**

**Table 1.** PC samples generated in extruder and PC types.

| Sample | PC1 | PC2 |
|---|---|---|
| *1* | 100% | 0 |
| *2* | 80 % | 20% |
| *3* | 60 % | 40% |
| *4* | 40 % | 60% |
| *5* | 20 % | 80% |
| *6* | 0 % | 100% |
| *PC1* | Branched bisphenol A polycarbonate homopolymer; produced vis melt polymerization; $M_w$ of about 41000 g/mol as determined by GPC using polystyrene standards; fries level about 350 ppm; BPA-Phenol endcapped. | |
| *PC2* | Linear bisphenol A polycarbonate homopolymer; produced via interfacial polymerization; $M_w$ of about 42000 g/mol as determined by GPC using polystyrene standards; p-cumylphenol endcapped | |

The melt volume flow rate (MVR), Fries content, and End cap ratios of PC1 (melt) and PC2 (interfacial) are presented in **Table 2.**

**Table 2.** Properties of PC1 (melt) and PC2 (interfacial) at MVR of 26 $cm^3$/10 min (with lower specification limit of 23.5 $cm^3$/10 min and upper specification limit of 28.5 $cm^3$/10 min).

| Grade | Mw (g/mol) | Fries | EC (%) | EC% (PhOH) | EC% (BPA) | EC% (PCP) |
|---|---|---|---|---|---|---|
| **PC1** | **40300** | **350** | **90** | **90** | **10** | **0** |

(continued)

| Grade | Mw (g/mol) | Fries | EC (%) | EC% (PhOH) | EC% (BPA) | EC% (PCP) |
|---|---|---|---|---|---|---|
| PC2 | 41000 | 0 | 100 | 0 | 0 | 100 |

The impact behavior (IZOD Notched Impact ISO 180-1A) was evaluated, for the samples in **Table 1** generated in the EMT extruder and according to the procedure described in the ISO norm: at 3 mm and at 7 different temperatures. The results for the samples tested are shown in the FIG. 1 for MVR 26 $cm^3$/10min (1.2 Kg at 300°C ISO 1133).

FIGs. 2 and 3 illustrate the effect of introducing the interfacial polycarbonate with different representations. FIG. 2 provides a graphical representation of the performance the impact performance as a function of the temperature for varying amounts of the PC1 melt PC resin. FIG. 3 provides a graphical representation of the impact performance observed at varying temperatures as a function of the amount of bulky endcap groups (bisphenol A, BPA and p-cumyl phenol, PCP) present in the sample. Table 3 shows the impact performance of samples with varying amounts of melt polycarbonate.

**Table 3.** Average Impact performance of sample blends at different amounts of melt PC1.

| PC2(%) | PC1 (%) | Bulky EC% | 23 °C | 10 °C | 5 °C | 0 °C | T - 5 °c | T -10 °C | T-20 °C |
|---|---|---|---|---|---|---|---|---|---|
| 0 | 100 | 14.7 | 50.9 | 37.8 | 13.2 | 13.8 | 12.2 | 11.6 | 10.2 |
| 20 | 80 | 31.1 | 57.3 | 34.7 | 26.3 | 15.0 | 13.5 | 13.0 | 11.1 |
| 40 | 60 | 47.8 | 56.3 | 44.6 | 46.8 | 34.5 | 19.6 | 13.4 | 12.0 |
| 60 | 40 | 64.9 | 57.0 | 51.4 | 54.1 | 38.5 | 31.3 | 23.6 | 12.3 |
| 80 | 20 | 82.3 | 56.2 | 55.6 | 52.9 | 49.2 | 43.3 | 23.0 | 13.5 |
| 100 | 0 | 100.0 | 57.8 | 55.1 | 50.1 | 49.7 | 39.9 | 23.4 | 12.9 |

As shown in FIG. 2, generally higher impact performance was observed with larger amounts of interfacial PC2 present in the sample. As shown in FIG. 3, the impact strength observed was plotted against the percentage of bulky end groups in the blend with or without interfacial PC2 resin present in the sample. The graph allows a determination of the minimum amount of interfacial PC2 resin in the blend needed to achieve an impact performance comparable to that of an interfacial PC resin. In certain aspects, at least 40% interfacial PC2 resin (47.8% bulky endgroups) is included in the blend formulation to achieve the same ductility for the melt material at 0 °C, 5 °C, and 10 °C. For lower temperatures, e.g., -5 °C and - 10 °C, the impact performance level increased with the addition of interfacial PC2 until the interfacial resin was present in an amount of about 60%.

FIGs. 4-7 further illustrate the effect on the impact performance as a result of the addition of the interfacial polycarbonate resin to the melt polycarbonate resin at the temperatures 10 °C, 5 °C, 0 °C, and -5 °C. FIGs. 4 and 5 present the impact performance of samples at 10 °C and 5 °C, respectively. Curves have been extrapolated from the data points for impact performance. As shown, up to about 70% of the interfacial polycarbonate can be added to the melt polycarbonate without affecting the impact performance of the melt resin, keeping the impact performance over a desirable 40 $kJ/m^2$. FIGs. 6 and 7 present the impact performance of samples at 0 °C and -1 °C, respectively. Here, for example, at 0 °C, up to 45 wt. % of the interfacial polycarbonate may be added to the melt resin to while maintaining a desired impact strength of greater than about 40 $kJ/m^2$.

Unexpectedly, the observed impact performance was also shown to exceed calculated impact performance. A mathematical formula used to calculate impact performance is as follows:

$$\text{Impact} = (\text{wt. \%}_{\text{PCmelt}} * \text{impact}_{\text{PCmelt}}) + (\text{wt. \%}_{\text{PCinterfacial}} * \text{impact}_{\text{PCinterfacial}})$$

As an example, at 5 °C, the observed impact at 50 wt.% interfacial PC and 50 wt. % melt PC was 45 $kJ/m^2$. According to the formula, the calculated impact value based on the linear weight combination of values of the resins would have been 32 $kJ/m^2$ [or, (0.5 * 52) + (0.5 * 12)]. Thus, the observed value of 45 $kJ/m^2$ compared to the calculated value of 32 $kJ/m^2$ represented an increase in the impact performance of about 40%.

According to these results it is possible to conclude that following a blend strategy with the melt and interfacial resin has a positive effect in the impact performance of the final resin compared with the impact behavior of the standard melt polycarbonate. This strategy can be used in order to produce materials with better impact performance properties at low temperature for low viscosity grades by blending the two types of resin (e.g., 20-30 $cm^3$/10 min at 1.2 kg (300 °C)).

It will be apparent to those skilled in the art that various modifications and variations can be made in the present

disclosure without departing from the scope or spirit of the disclosure. Other aspects of the disclosure will be apparent to those skilled in the art from consideration of the specification and practice of the disclosure disclosed herein. It is intended that the specification and examples be considered as exemplary only, with a true scope and spirit of the disclosure being indicated by the following claims.

The patentable scope of the disclosure is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A composition comprising:

   a melt polycarbonate resin derived from diphenyl carbonate; and
   an interfacial polycarbonate resin mixed with the melt polycarbonate resin,
   wherein the composition exhibits a melt volume rate of between about 20 $cm^3$/10 minutes and about 30 $cm^3$/10 minutes at 1.2 kg and 300 °C,
   wherein an article formed from the composition exhibits an IZOD Notched Impact performance that is greater than an IZOD Notched Impact performance of an article formed from a comparator composition consisting essentially of the melt polycarbonate resin within the ductile to brittle transition temperature range of the composition and the comparator composition.

2. A composition comprising:

   a melt polycarbonate resin derived from diphenyl carbonate; and
   an interfacial polycarbonate resin mixed with the melt polycarbonate resin,
   wherein the composition exhibits a melt volume rate of between about 20 $cm^3$/10 minutes and about 30 $cm^3$/10 minutes at 1.2 kg and 300 °C,
   wherein an article formed from the composition exhibits an IZOD Notched Impact performance that is greater than an IZOD Notched Impact performance of an article formed from a comparator composition consisting essentially of the melt polycarbonate resin at a temperature in the range of about -20 °C and about 10 °C.

3. The composition of any one of claims 1-2, wherein the article formed from the composition is transparent.

4. The composition of any one of claims 1-3, wherein the interfacial polycarbonate resin has an endcap of p-cumyl phenol, p-tertbutyl phenol, phenol, or a combination thereof, wherein preferably the endcap is p-terbutyl phenol.

5. The composition of any one of claims 1-4, wherein the composition exhibits a melt volume rate of between about 21 $cm^3$/10 minutes and about 26 $cm^3$/10 minutes at 1.2kg and 300 °C.

6. The composition of any one of claims 1-4, wherein the composition exhibits a melt volume rate of 26 $cm^3$/10 minutes at 1.2 kg and 300 °C.

7. The composition of any one of claims 1-6, wherein the article formed from the composition exhibits an IZOD Notched Impact performance that is greater than an IZOD Notched Impact performance of an article formed from a comparator composition consisting essentially of the melt polycarbonate resin at about -5 °C.

8. The composition of any one of claims 1-6, wherein the article formed from the composition exhibits an IZOD Notched Impact performance that is greater than an IZOD Notched Impact performance of an article formed from a comparator composition consisting essentially of the melt polycarbonate resin at a temperature between about -15 °C and about 0 °C.

9. The composition of any one of claims 1-6, wherein the article formed from the composition exhibits an IZOD Notched Impact performance that is greater than an IZOD Notched Impact performance of an article formed from a comparator composition consisting essentially of the melt polycarbonate resin at a temperature between about -10 °C and about -5 °C.

10. The composition of any one of claims 1-9, wherein the composition comprises about 20 wt% to about 80% of the melt polycarbonate resin relative to 100 wt% of all polycarbonate resin in the composition.

11. The composition of any one of claims 1-9, wherein the composition comprises about 20 wt% of the melt polycarbonate resin and about 80% of the interfacial polycarbonate resin relative to 100 wt% of all polycarbonate resin in the composition.

12. The composition of any one of claims 1-9, wherein the composition comprises about 40 wt% of the melt polycarbonate resin and about 60% of the interfacial polycarbonate resin relative to 100 wt% of all polycarbonate resin in the composition.

13. The composition of any one of claims 1-9, wherein the composition comprises about 60 wt% of the melt polycarbonate resin and about 40% of the interfacial polycarbonate resin relative to 100 wt% of all polycarbonate resin in the composition.

14. The composition of any one of claims 1-9, wherein the composition comprises about 80 wt% of the melt polycarbonate resin and about 20% of the interfacial polycarbonate resin relative to 100 wt% of all polycarbonate resin in the composition.

15. A method of making the composition of any one of claims 1-14.

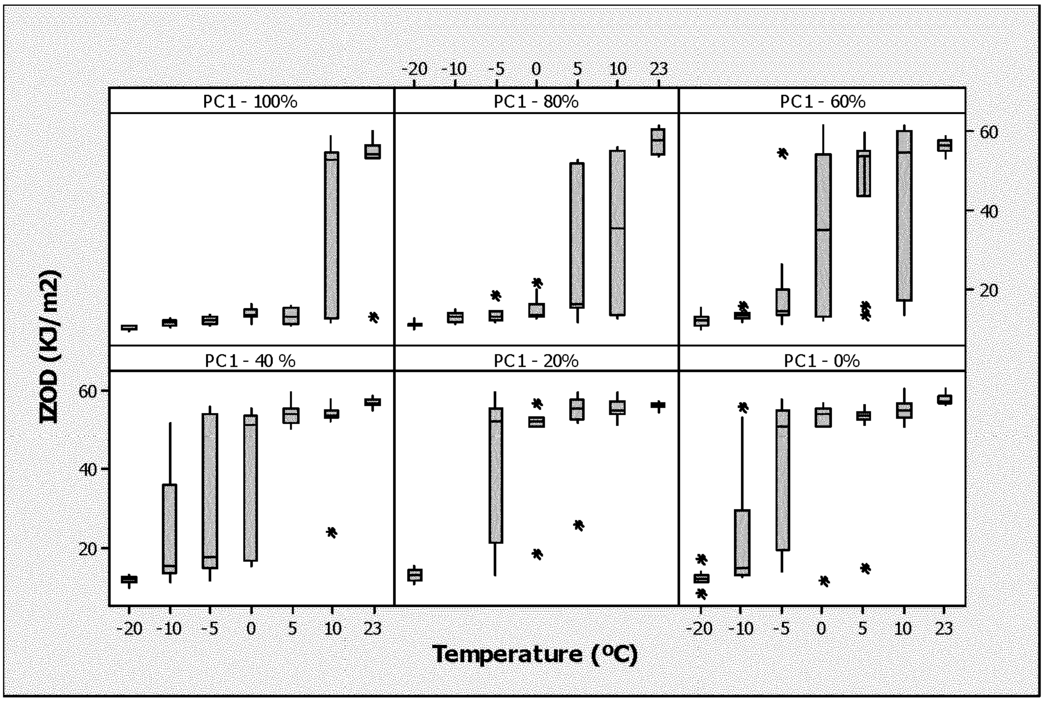
PC1 - 100%
PC1 - 80%
PC1 - 60%
PC1 - 40 %
PC1 - 20%
PC1 - 0%
IZOD (KJ/m2)
Temperature (ºC)
-20 -10 -5 0 5 10 23
20
40
60

FIG. 1

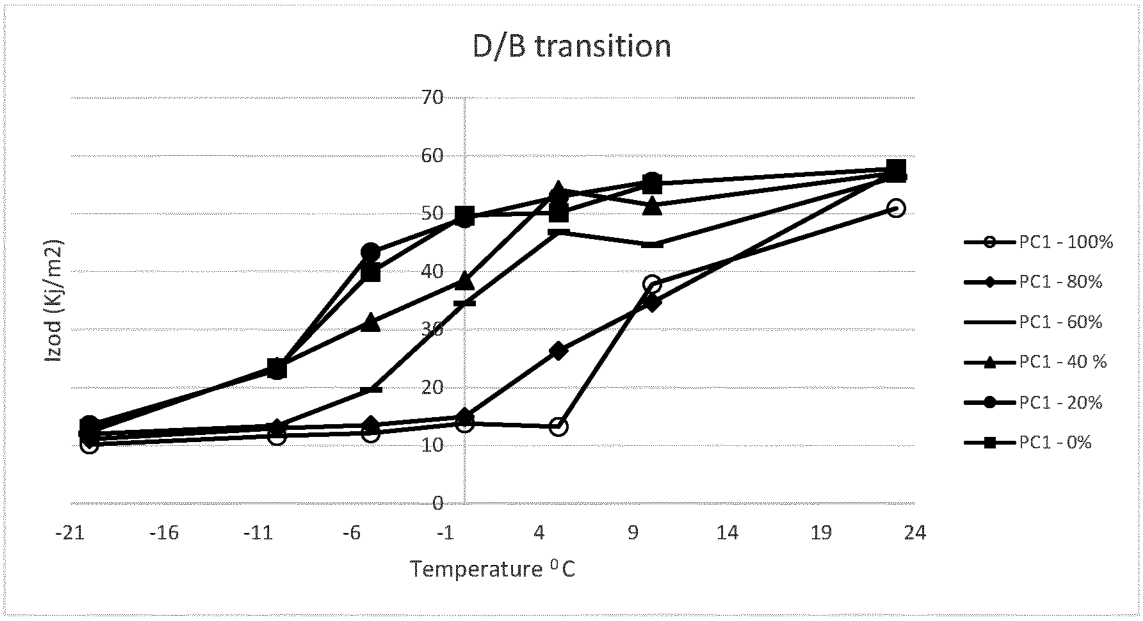
D/B transition
70
60
50
40
30
20
10
0
Izod (Kj/m2)
-21
-16
-11
-6
-1
4
9
14
19
24
Temperature ⁰C
PC1 - 100%
PC1 - 80%
PC1 - 60%
PC1 - 40 %
PC1 - 20%
PC1 - 0%

FIG. 2

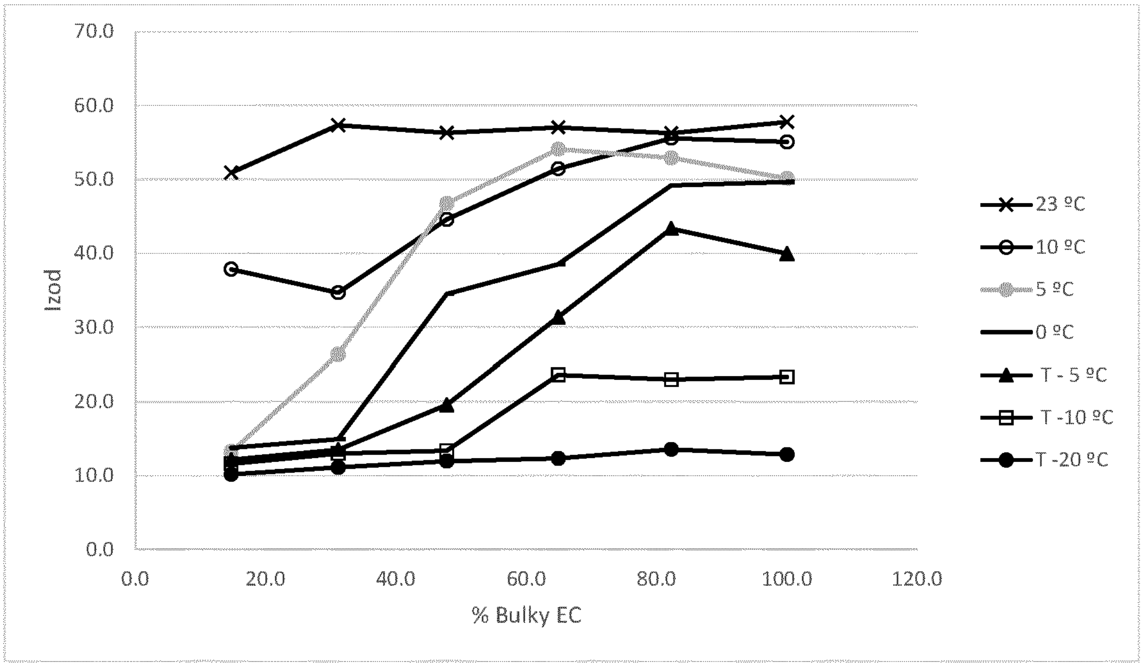
70.0
60.0
50.0
40.0
30.0
20.0
10.0
0.0
Izod
0.0
20.0
40.0
60.0
80.0
100.0
120.0
% Bulky EC
23 ºC
10 ºC
5 ºC
0 ºC
T - 5 ºC
T -10 ºC
T -20 ºC

FIG. 3

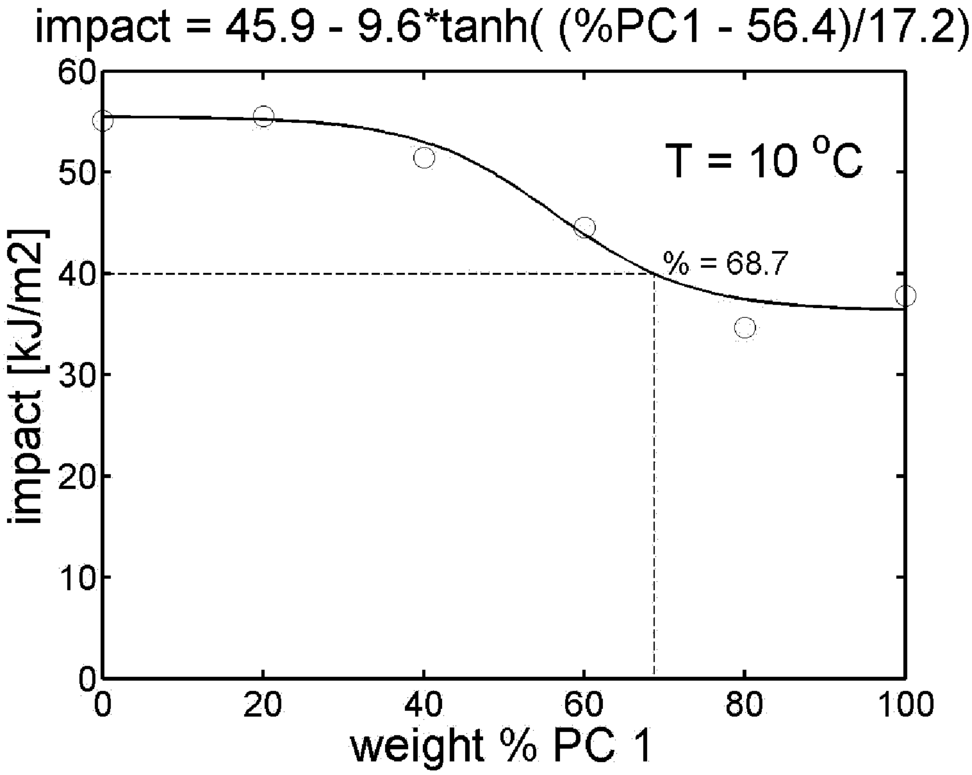
impact = 45.9 - 9.6*tanh( (%PC1 - 56.4)/17.2)
T = 10 °C
% = 68.7
impact [kJ/m2]
0
10
20
30
40
50
60
0
20
40
60
80
100
weight % PC 1

FIG. 4

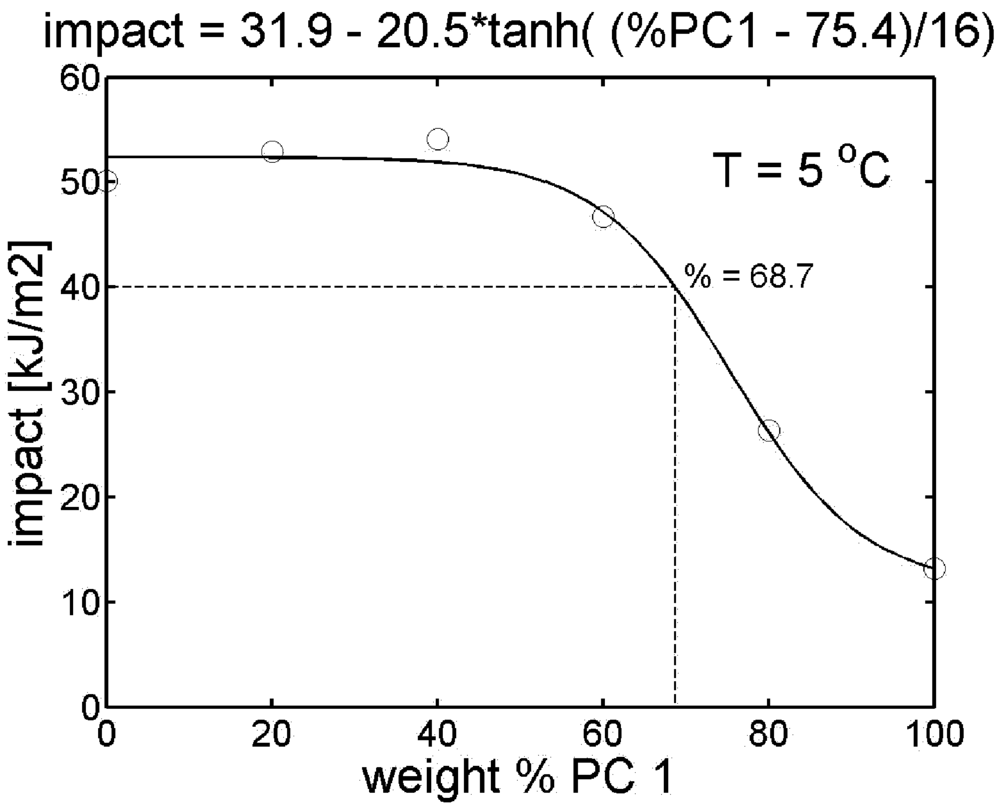
impact = 31.9 - 20.5*tanh( (%PC1 - 75.4)/16)
T = 5 °C
% = 68.7
impact [kJ/m2]
0
10
20
30
40
50
60
0
20
40
60
80
100
weight % PC 1

FIG. 5

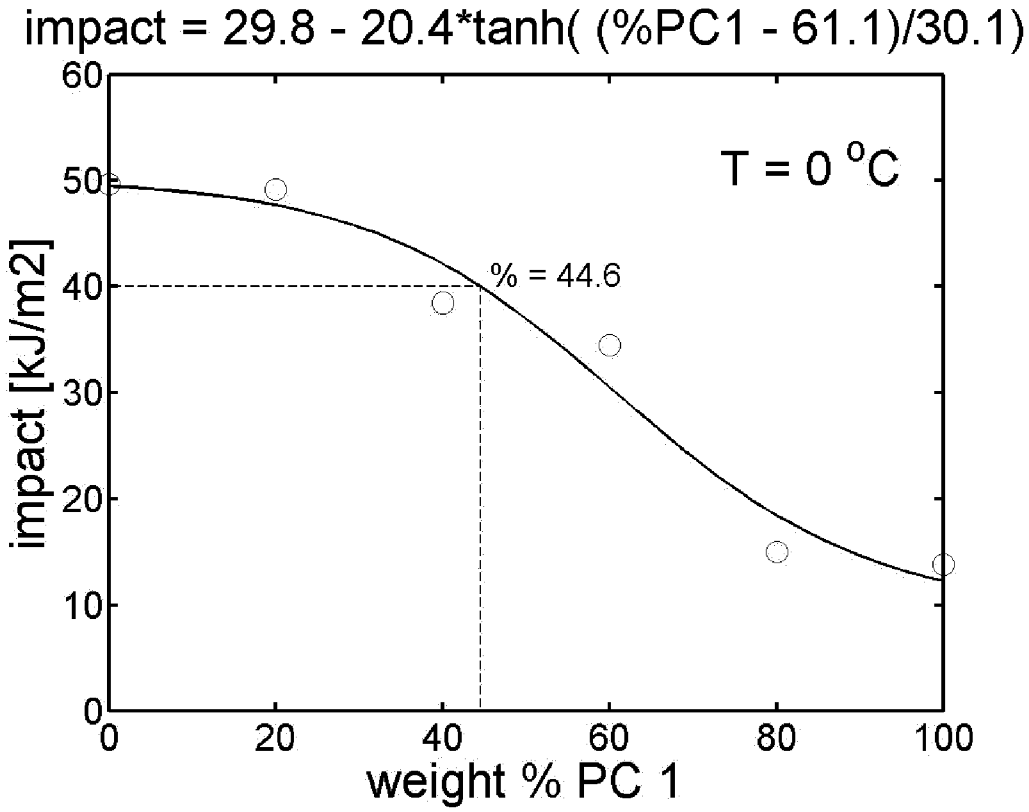
impact = 29.8 - 20.4*tanh( (%PC1 - 61.1)/30.1)
T = 0 °C
% = 44.6
impact [kJ/m2]
weight % PC 1
0
10
20
30
40
50
60
0
20
40
60
80
100

FIG. 6

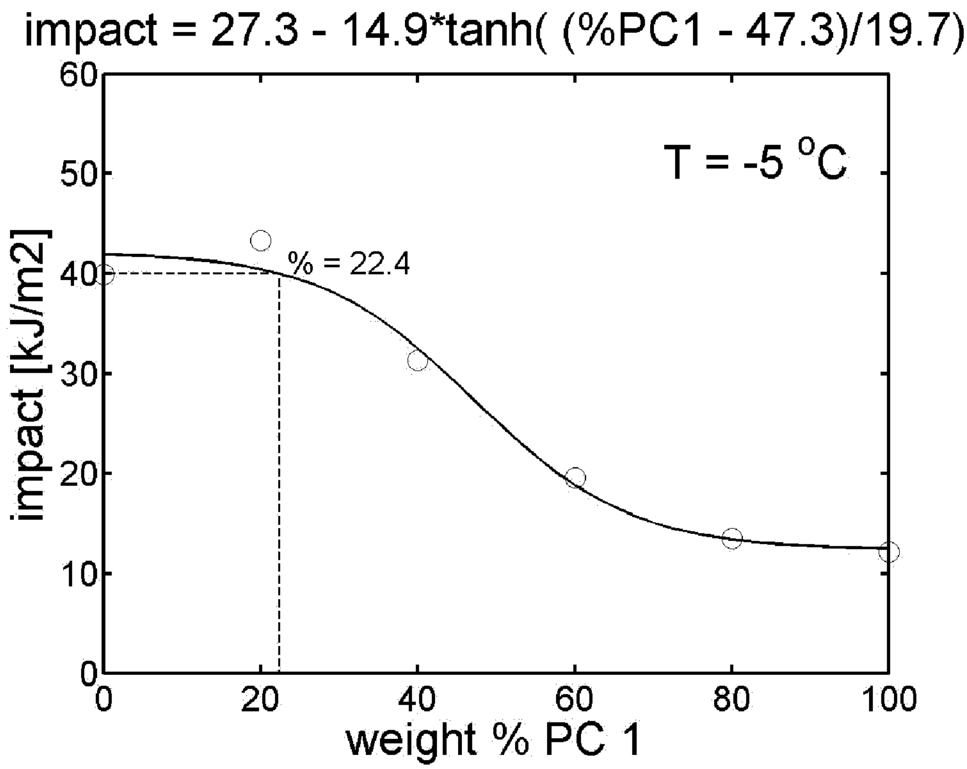
impact = 27.3 - 14.9*tanh( (%PC1 - 47.3)/19.7)
T = -5 °C
% = 22.4
impact [kJ/m2]
weight % PC 1
0
10
20
30
40
50
60
0
20
40
60
80
100

FIG. 7

Europäisches Patentamt
European Patent Office
Office européen des brevets

# EUROPEAN SEARCH REPORT

Application Number
EP 25 17 3141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/137500 A1 (GOOSSENS JOHANNES MARTINUS DINA [NL] ET AL) 3 June 2010 (2010-06-03) * paragraph [0077]; examples; table 5 * * abstract * ----- | 1-15 | INV. C08L69/00 |
| A | WO 2013/190474 A1 (SABIC INNOVATIVE PLASTICS IP [NL]) 27 December 2013 (2013-12-27) * abstract; tables * ----- | 1-15 | |
| | | | TECHNICAL FIELDS SEARCHED (IPC) |
| | | | C08L |

The present search report has been drawn up for all claims

1

| Place of search | Date of completion of the search | Examiner |
|---|---|---|
| The Hague | 11 August 2025 | Schlicke, Benedikt |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 17 3141

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2025

| Patent document cited in search report | Publication date | Patent family member(s) | Publication date |
|---|---|---|---|
| US 2010137500 A1 | 03-06-2010 | AT E548424 T1 | 15-03-2012 |
| | | CN 101802093 A | 11-08-2010 |
| | | EP 2152808 A1 | 17-02-2010 |
| | | KR 20100017703 A | 16-02-2010 |
| | | US 2010137500 A1 | 03-06-2010 |
| | | WO 2008136839 A1 | 13-11-2008 |
| WO 2013190474 A1 | 27-12-2013 | CN 104428343 A | 18-03-2015 |
| | | EP 2864391 A1 | 29-04-2015 |
| | | KR 20150023573 A | 05-03-2015 |
| | | US 2013345347 A1 | 26-12-2013 |
| | | WO 2013190474 A1 | 27-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5504177 A
- US 6066700 A
- US 5959065 A
- US 7365149 B
- US 7547799 B
- US 7619053 B
- US 7671165 B
- US 4217438 A
- US 3635895 A
- US 4001184 A
- US 5151491 A
- US 5142018 A


### Non-patent literature cited in the description


- *CHEMICAL ABSTRACTS*, 82091-12-1